# EUROPEAN PATENT SPECIFICATION

(11) **EP 1 242 368 B1**
(45) Date of publication and mention of the grant of the patent: **15.09.2004**
(21) Application number: 00986437.2
(22) Date of filing: 14.12.2000
(51) Int. Cl.: C07C 269/04, C07C 271/28

(54) **PROCESS FOR THE SYNTHESIS OF POLYCARBAMATES**
VERFAHREN ZUR HERSTELLUNG VON POLYCARBAMATEN
PROCEDE DE SYNTHESE DES POLYCARBAMATES

(30) Priority: 27.12.1999 IN BO096099
(43) Date of publication of application: 25.09.2002
(73) Proprietor: HUNTSMAN INTERNATIONAL LLC, Salt Lake City, Utah 84108 (US)
(72) Inventor: CHAUDHARI, Raghunath, Vitthal, Pune 411 008 (IN); KELKAR, Ashutosh, Anant, Pune 411 038 (IN); GUPTE, Sunil, Purushottam, Pune 411 008 (IN); DIVEKAR, Sunil, Sadashiv, Pune 411 008 (IN); GANAPATHY, Subramanian, Pune 411 008 (IN)
(74) Representative: Moens, Marnix Karel Christiane
(86) International application number: PCT/US2000/034103
(87) International publication number: WO 2001/047871

(56) References cited:
- EP-A- 0 359 519
- US-A- 4 260 781
- US-A- 4 375 000
- US-A- 5 194 660

## Description

### Field of Invention

This invention relates to a method for the preparation of polymeric carbamates by oxidative carbonylation of polymeric amino compounds. More particularly, it relates to an industrially advantageous method of producing polymeric MDU (methylene diphenyl diurethane) in high selectivity by a direct carbonylation method. This route provides a non-phosgene method for the preparation of polymeric carbamates.

### Background of the Invention

Conventionally, aromatic diisocyanates are prepared by phosgenation of the corresponding diamino compounds. The use of phosgene results in the generation of hydrochloric acid as a side product, which is the cause of severe corrosion. Considering the environmental hazard and corrosion problems in the phosgenation route, it is important to develop a route without using phosgene.

MDI is an important starting material for the production of polyurethanes which are useful as elastomers, artificial leather, coatings, spandex fibers etc. Diphenyl methane dicarbamates are useful precursors for the preparation of diphenylmethane diisocyanates (MDI).

Various methods for the preparation of alkyl and aromatic carbamates by carbonylation of corresponding amino compounds with carbon monoxide and oxygen using Group VIII metal catalysts have been reported (US-A 5,502,241; 5,194,660; 4,694,097 and 4,242,520; EP-A 83096).

Asahi Chemicals have proposed a process for the preparation of 4,4'-MDU (US-A 4,552,974; 4,230,877; 4,319,018; GB-A 2054584; JP-A 12357/81) by condensation of ethyl N-phenyl carbamate with formaldehyde in the presence of acids, such as mineral acids or organic sulfonic acids. This requires relatively severe reaction conditions.

Recently, Valli and Alper have published a work on oxidative carbonylation of various aliphatic diamines to aliphatic diurethanes using Pd-clay/NaI catalyst system (Organomett. 14, 81 (1995)).

Oxidative carbonylation of aniline to the corresponding aromatic urethane has already been disclosed in Catalysis Letters (1994). 25, p.361-364.

However, none of the prior art documents report catalytic conversion of polymeric amino compounds to their corresponding carbamate derivatives by a clean and environmentally benign route.

US 4,375,000 discloses a method for preparing a polymeric carbamate by reaction of a polymeric amine with an alkyl carbamate.

The present invention is concerned with the use of a catalytic route for the conversion of aromatic polyamino compounds to polycarbamates via oxidative carbonylation. Such a route would eliminate the use of toxic phosgene and associated corrosion problems leading to an environmentally benign process.

### Summary of the Invention

The present invention relates to a clean catalytic process for producing polymeric carbamate derivatives in a single step, wherein an aromatic polyamine reacts with carbon monoxide, oxygen and hydroxyl containing organic compounds in the presence of a transition metal catalyst and a halogenide an iodide promoter. Usually, the reaction is carried out in a liquid phase at 60 to 300°C and pressures of 10 bar to 100 bar. The catalyst compositions described herein comprise a Group VIII metal (e.g. palladium, platinum, nickel, rhodium, ruthenium or cobalt) or a complex thereof, or a lanthanide metal, particularly cerium. They are used in combination with a halogenide, e.g. an iodide, source as promoter, as illustrated by various alkali metal iodides.

Any aromatic polyamine may be employed in the present invention, specifically polymeric DADPM (DiAmino DiPhenyl Methane). Other essential reagents in the method of this invention are an oxidising agent, such as oxygen, and carbon monoxide, which react with the polymeric amine and hydroxy compound to form the desired polymeric carbamate.

The end product formed in the present invention is the compound represented by formula I :

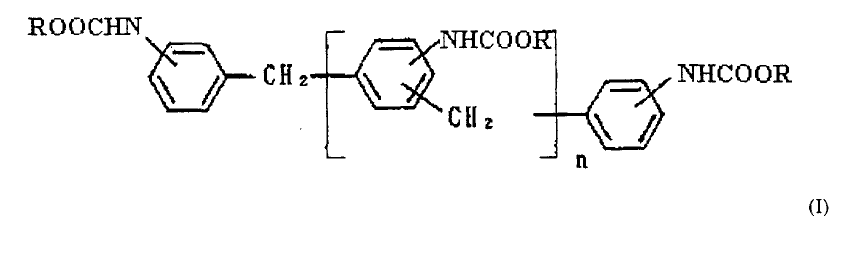

Wherein n is greater than 0, suitably 1-20, preferably 1-10; R is an alkyl group having 1-15 carbon atoms, preferably 1-6 carbon atoms, aromatic groups or an alicyclic group having 3-30 carbon atoms, preferably 5-10 carbon atoms. Preferred examples of R groups are methyl, ethyl, propyl (n- or iso-), butyl (n- or iso-), pentyl (n- and various isomers), hexyl (n- and various isomers), alicyclic groups such as cyclopentyl and cyclohexyl and aromatic groups such as phenyl, or halogenated phenyl.

Representative examples of the compound in the formula I includes polymeric dimethyl diphenylmethane dicarbamates, diethyl diphenylmethane dicarbamates, dipropyl diphenylmethane dicarbamates.

Suitable hydroxyl compounds include aliphatic or aromatic, cyclic or alicyclic alcohols such as, for example, methanol, ethanol, n-propanol, isopropanol, pentanol, hexanol, 3,3-dimethyl-2-butanol, 2-propanol, N,N-dimethyl- ethanolamine, 1-methoxy-2-propanol, 2-methoxyethanol, 1-ethoxycyclo-propanol, 2-isopropoxyethanol, 1,3-dimethoxy-2-propanol, 1,1-dimethoxy- ethanol, 2-methoxy-1-propanol, 2-methoxy-3-propanol, dimethyloxime, 1,3-dimethoxy-2-propanol , benzyl alcohol, phenol, hydroxylamines, halogenated alcohols such as hexafluoroisopropanol or trifluoroethanol, halogenated phenols such as ortho-chlorophenol, para-chlorophenol, ortho-fluorophenol, para-fluorophenol and the like.

An essential constituent of the catalyst composition employed according to the invention is one of the Group VIII-B metals, preferably palladium, or a compound thereof. Thus, palladium black or elemental palladium deposited on zeolite, carbon and oxide supports such as alumina, silica are suitable, as well as palladium compounds such as halides, and complexes with such compounds as carbon monoxide, amines, phosphines and olefins. The catalytic material also contains a halogenide source, preferably an iodide source. It may be an alkali metal iodide or alkyl iodide.

Carbon monoxide employed in the process of the present invention may be pure gaseous carbon monoxide, but may also contain impurities such as nitrogen and carbon dioxide. An impurity content of less than 10% v/v does not affect the reaction pattern and from industrial viewpoint, it may be advantageous to use carbon monoxide with small amounts of impurities. Carbon monoxide is employed in an amount of at least 1 mole per amino group of the polymeric amine compound. A more preferred amount of carbon monoxide is from 2 to 100 moles per amino group of the polymeric amine compound.

The oxidising agent used in the process of the invention may be pure oxygen, a gas containing oxygen such as air or an organic nitro compound or a mixture thereof. Oxygen however is preferred. The process also tolerates the employment in some cases of an oxygen containing gas, which additionally contains other non-interfering gases, such as argon, nitrogen or carbon dioxide.

The organic hydroxyl compound, which forms one of the reactants, can also function as a solvent. However, where necessary, other solvents, which do not affect the reaction adversely (i.e. inert solvents), may also be used. Exemplary of such solvents are aromatic hydrocarbons such as benzene, toluene, xylene, monochlorobenzene, orthodichlorobenzene, and nitriles such as acetonitrile and benzonitrile, ethers such as tetrahydrofuran and 2-dioxan, ketones such as acetone and methyl ethyl ketone, amides such as N,N' dimethyl formamide and N,N' dimethyl acetamide and esters such as ethyl acetate and ethyl benzoate.

The component of the catalyst which acts as a precursor can comprise a soluble compound of a transition metal (Group VIII-B) supported an a suitable carrier. Of the transition metals, palladium is especially preferred. Specific examples of the catalyst precursors include Palladium black, supported palladium catalysts such as Pd/C, Pd/Al₂O₃, Pd/ZSM5, Pd/CaCO₃ and the like. Pd black, which has been prepared by various reducing agents, such as hydrazine hydrate, sodium formate, formaldehyde, sodium borohydride, LiAlH₄, and H₂, can be used.

Soluble Pd compounds that can be used are : PdCl₂, PdBr₂, Pd(NO₃)₂, Pd(OAc)₂, Pd oxalate, [Pd(NH₃)₄]X₂, PdL₂X, Pd(CO)X, wherein X is Cl, Br or I, and L is triphenyl phosphine, pyridine, isoquinoline, tributyl phosphine, benzonitrile and the like.

The halogen-containing promoter can be selected from alkali metal halides, alkaline earth metal halides, quaternary ammonium halides, oxo acids of halogen atom and their salts, and complexes containing halogen ions, organic halides and halogen molecules. However, of all the halogen compounds, which act as promoters, those compounds containing iodine are particularly preferred. These include KI, NaI, LiI, CsI, tetrabutyl ammonium iodide, iodine and the like.

The oxidative carbonylation reaction of the present invention can be carried out in a temperature range of 80-350°C, more preferably between 120-250°C. However, it has been found that temperature is an important factor in obtaining a good yield of carbamate derivatives. The carbonylation is carried out under a CO partial pressure of about 5-6000 psig, more preferably between 100-1500 psig. The partial pressure of O₂ is employed between 5-1000 psig, more preferably between 10-300 psig. The ratio of CO to O₂ used in this process is an important factor and activity and selectivity of the catalyst was found to be drastically affected if the CO to O₂ ratio was varied. The ratio of CO to O2 in the reactor can be in the range of 1:1 to 50:1, preferably in the range of 5:1 to 20:1.

In giving effect to the reaction of the present invention, it has been found convenient to employ 1 mole of catalyst per 5-8000 moles of amine functionality. More preferred range comprises 1 mole of catalyst per 100-500 moles of amine functionality. The ratio of iodide promoter to metal is in the range of about 0.1 to 50, and more preferably between 0.5 to 15.

The amount of organic hydroxyl compound employed is at least 1 mole per amine group of polymeric amine compound. However, it is more preferable to use 3-100 moles of the hydroxyl group per amino group of polymeric amine compound.

The invention will now be described in detail in the following examples, which should not however be considered to limit the scope of the inventive process. The invention is illustrated by the following examples

### Example 1

Polymeric methylene diphenyl urethane was prepared by charging the following components into a 50 ml high-pressure stirred autoclave.

| | |
|---|---|
| polymeric DADPM | 0.8 g |
| Pd-ZSM-5(10%) | 0.08 g |
| Sodium iodide (NaI) | 0.008 g |
| and excess of ethyl alcohol. | 20 ml |

The autoclave was sealed, flushed twice with carbon monoxide, pressurised with 744 psig of carbon monoxide and 56 psig of oxygen resulting in a total pressure of 800 psig at room temperature. The reaction was carried out at 190°C (1000 psig) for two hours with constant vigorous stirring. The progress of the reaction was monitored by recording the pressure drop, and the gases were repressurised in a 2:1 ratio of carbon monoxide to oxygen as necessary. After 2 hr., the reactor was cooled and discharged. The liquid portion was filtered from the insoluble material. The solvent ethyl alcohol was evaporated completely under vacuum. A sticky solid product, weighing about 1.09 g was thus obtained. The product was analysed by IR, ¹H and ¹³C NMR and proved to be more than 95% pure which results in an estimated yield of 75-80%.

### Example 2

The procedure of example 1 was repeated except that the reactor used was 300 ml instead of 50 ml. The following components were charged to the reactor :

| | |
|---|---|
| polymeric DADPM | 6.4 g |
| Pd-ZSM-5(10%) | 0.64 g |
| Sodium iodide (NaI) | 0.064 g |

and excess of ethyl alcohol.

The yield obtained of the sticky solid product was about 10.6 g. Estimated yield of carbamate based on weight and ¹³C NMR analysis is 75-80%. About 20% of unreacted aromatic amine functions were observed.

### Example 3

The procedure of example 1 was repeated except that the catalyst 1% Pd-ZSM-% was employed instead of 10% Pd-ZSM-%. The components charged were :

| | |
|---|---|
| polymeric DADPM | 0.8 g |
| Pd-ZSM-5(1%) | 0.08 g |
| Sodium iodide (NaI) | 0.008 g |

and excess of ethyl alcohol.

An insoluble residue of about 0.44g was obtained together with a soluble fraction which based on ¹³C NMR proved to be pure carbamate.

### Example 4

The procedure of example 1 was repeated except that the catalyst Pd metal was employed instead of 10%Pd-ZSM-5. The components charged were :

| | |
|---|---|
| polymeric DADPM | 0.8 g |
| Pd-metal | 0.004 g |
| Sodium iodide (NaI) | 0.008 g |

and excess of ethyl alcohol.

An insoluble residue of about 0.44g was obtained together with a soluble fraction which based on ¹³C NMR proved to contain carbamate.

### Example 5

The procedure of example 1 was repeated except that the catalyst Pd acetate was employed instead of 10%Pd-ZSM-5. The components charged were :

| | |
|---|---|
| polymeric DADPM | 0.8 g |
| Pd(OAc)₂ | 0.008 g |
| Sodium iodide (NaI) | 0.008 g |

and excess of ethyl alcohol.

An insoluble residue of about 0.02g was obtained together with a soluble fraction which based on ¹³C NMR proved to contain carbamate.

### Comparative example 1

The procedure of example 1 was repeated except that 4,4' DADPM was used instead of polymeric DADPM as the starting compound. An insoluble residue of 0.12 g was obtained together with a soluble fraction containing 61 % 4,4' diethyl carbamate and 2.4 % 4-amino-4'-ethylcarbonylamino-diphenyl methane (monocarbamate), both measured by HPLC.

### Comparative example 2

The procedure of example 2 was repeated except that the amount of start material used was halved and that 4,4' DADPM was used instead of polymeric DADPM as the starting compound. An insoluble residue of 1.12 g was obtained together with a soluble fraction containing 49.4 % 4,4' diethyl carbamate and 2.4 % 4-amino-4'-ethylcarbonylaminodiphenyl methane (monocarbamate), both measured by HPLC.

### Comparative example 3

The procedure of example 3 was repeated except that 4,4' DADPM was used instead of polymeric DADPM as the starting compound. An insoluble residue of 0.106 g was obtained together with a soluble fraction containing 49.5 % 4,4' diethyl carbamate and 7.92 % 4-amino-4'-ethylcarbonylamino-diphenyl methane (monocarbamate), both measured by HPLC.

### Comparative example 4

The procedure of example 4 was repeated except that 4,4' DADPM was used instead of polymeric DADPM as the starting compound and that the reaction temperature was 170°C. An insoluble residue of 0.32 g was obtained together with a soluble fraction containing 24.1 % 4,4' diethyl carbamate and 6.4 % 4-amino-4'-ethylcarbonylamino-diphenyl methane (monocarbamate), both measured by HPLC.

### Comparative example 5

The procedure of example 5 was repeated except that 4,4' DADPM was used instead of polymeric DADPM as the starting compound. An insoluble residue of 0.05 g was obtained together with a soluble fraction containing 52.3 % 4,4'diethyl carbamate and 2.7 % 4-amino-4'-ethylcarbonylamino-diphenyl methane (monocarbamate), both measured by HPLC.

## Claims

1. A method for preparing a polymeric carbamate compound which comprises reacting a polymeric amine with carbon monoxide, an oxidising agent and an organic hydroxyl compound in the presence of a catalyst system comprising of
a) a precursor containing a Group VIII or lanthanide metal and
b) at least one halogen containing promoter effective to promote the said reaction.

2. A method according to claim 1 wherein the polymeric amine compound is polymeric diphenylmethane diamine.

3. A method according to claim 1 or 2 wherein the metal is palladium or cerium.

4. A method according to any one of claims 1-3 wherein the precursor is selected from the group consisting of Pd black, Pd/C, Pd/Al₂O₃, Pd/CaCO₃, Pd/ZSM5, PdCl₂, PdBr₂, PdI₂, Pd(NO₃)₂, Pd(OAc)₂, Pd oxalate, [Pd(NH₃)₄]X₂, PdL₂X, Pd(CO)X, wherein X is Cl, Br or I.

5. A method according to any one of claims 1-4 wherein the oxidising agent is oxygen or air.

6. A method according to any one of claims 1-5 wherein said halogen containing promoter contains iodine.

7. A method according to claim 6 wherein the halogen containing promoter is selected from the group consisting of NaI, LiI, CsI, tetra butyl ammonium iodide, tetra heptyl ammonium iodide, and iodine.

8. A method according to any one of claims 1-7 wherein the proportion of palladium is about 1 mole per 100-500 moles of amino group in the polymeric amine compound.

9. A method according to any one of claims 1-8 wherein the ratio of iodide promoter to catalyst precursor is in the range of about 0.5-15.

10. A method according to any one of claims 1-9 wherein the temperature is about 100° to 300°C temperature is present.

11. A method according to any one of claims 1-10 wherein the pressure is about 10 to 100 bar.

12. A method according to any one of claims 1-11 wherein the reaction is effected in the absence of an inert solvent.

13. A method according to any one of claims 1-11 wherein the reaction is effected in the presence of an inert solvent.

14. A method according to claim 13 wherein said solvent is selected from aromatic hydrocarbons, nitriles, ethers, ketones, amides and esters.

15. A method according any one of claims 1-14 wherein oxygen is present in the reactor and the CO:O₂ ratio in the range of about 5:1 to 20:1.

## Patentansprüche

1. Ein Verfahren zur Herstellung einer polymeren Carbamatverbindung, das die Umsetzung eines polymeren Amins mit Kohlenmonooxid, einem Oxidationsmittel und einer organischen Hydroxylverbindung in Gegenwart eines Katalysatorsystems, das umfasst
a) einen Precursor, der ein Gruppe VIII- oder Lanthanoidmetall enthält, und
b) wenigstens einen halogenhaltigen Promotor, der die Reaktion wirksam fördern kann,
umfasst.

2. Ein Verfahren gemäß Anspruch 1, wobei die polymere Aminverbindung polymeres Diphenylmethandiamin ist.

3. Ein Verfahren gemäß Anspruch 1 oder 2, wobei das Metall Palladium oder Cer ist.

4. Ein Verfahren gemäß einem der Ansprüche 1-3, wobei der Precursor aus der Gruppe, die aus Pd-Schwarz, Pd/C, Pd/Al₂O₃, Pd/CaCO₃, Pd/ZSM5, PdCl₂, PdBr₂, PdI₂, Pd(NO₃)₂, Pd(OAc)₂, Pd-Oxalat, [Pd(NH₃)₄]X₂, PdL₂X, Pd(CO)X besteht, ausgewählt wird, wobei X -Cl, -Br oder -I ist.

5. Ein Verfahren gemäß einem der Ansprüche 1-4, wobei das Oxidationsmittel Sauerstoff oder Luft ist.

6. Ein Verfahren gemäß einem der Ansprüche 1-5, wobei der halogenhaltige Promoter Jod enthält.

7. Ein Verfahren gemäß Anspruch 6, wobei der halogenhaltige Promotor aus der Gruppe, die aus NaI, LiI, CsI, Tetrabutylammoniumiodid, Tetraheptylammoniumiodid und Jod besteht, ausgewählt wird.

8. Ein Verfahren gemäß einem der Ansprüche 1-7, wobei der Palladiumanteil ungefähr 1 mol pro 100-500 mol der Aminogruppe in der polymeren Aminverbindung ist.

9. Ein Verfahren gemäß einem der Ansprüche 1-8, wobei das Verhältnis des Jod-Promotors zum Katalysator-Precursor im Bereich von ungefähr 0.5-15 ist.

10. Ein Verfahren gemäß einem der Ansprüche 1-9, wobei die Temperatur ungefähr 100° bis 300°C ist.

11. Ein Verfahren gemäß einem der Ansprüche 1-10, wobei der Druck ungefähr 10 bis 100 bar ist.

12. Ein Verfahren gemäß einem der Ansprüche 1-11, wobei die Reaktion in Abwesenheit eines inerten Lösungsmittels ausgeführt wird.

13. Ein Verfahren gemäß einem der Ansprüche 1-11, wobei die Reaktion in Gegenwart eines inerten Lösungsmittels ausgeführt wird.

14. Ein Verfahren gemäß Anspruch 13, wobei das Lösungsmittel aus aromatischen Kohlenwasserstoffen, Nitrilen, Ethern, Ketonen, Amiden und Estern ausgewählt wird.

15. Ein Verfahren gemäß einem der Ansprüche 1-14, wobei Sauerstoff im Reaktor vorhanden ist und das CO:O₂-Verhältnis im Bereich von ungefähr 5:1 bis 20:1 ist.

## Revendications

1. Procédé pour la préparation d'un carbamate polymère, qui comprend la réaction d'une amine polymère avec du monoxyde de carbone, un agent oxydant et un composé hydroxylique organique en présence d'un système de catalyseur comprenant :
a) un précurseur contenant un métal du Groupe VIII ou faisant partie des lanthanides, et
b) au moins un promoteur halogéné efficace pour favoriser ladite réaction.

2. Procédé suivant la revendication 1, dans lequel l'amine polymère est la diphénylméthane-diamine polymère.

3. Procédé suivant la revendication 1 ou 2, dans lequel le métal est le palladium ou le cérium.

4. Procédé suivant l'une quelconque des revendications 1 à 3, dans lequel le précurseur est choisi dans le groupe consistant en noir de Pd, Pd/C, Pd/Al₂O₃, Pd/CaCO₃, Pd/ZSM5, PdCl₂, PdBr₂, PdI₂, Pd(NO₃)₂, Pd(OAc)₂, oxalate de Pd, [Pd(NH₃)₄]X₂, PdL₂X, Pd(CO)X, dans lesquels X représente Cl, Br ou I.

5. Procédé suivant l'une quelconque des revendications 1 à 4, dans lequel l'agent oxydant est l'oxygène ou l'air.

6. Procédé suivant l'une quelconque des revendications 1 à 5, dans lequel ledit promoteur halogéné contient de l'iode.

7. Procédé suivant la revendication 6, dans lequel le promoteur halogéné est choisi dans le groupe consistant en NaI, LiI, CsI, iodure de tétrabutylammonium, iodure de tétraheptylammonium et iode.

8. Procédé suivant l'une quelconque des revendications 1 à 7, dans lequel la proportion de palladium est d'environ 1 mole pour 100-500 moles de groupe amino dans l'amine polymère.

9. Procédé suivant l'une quelconque des revendications 1 à 8, dans lequel le rapport du promoteur iodure au précurseur de catalyseur est compris dans l'intervalle d'environ 0,5 à 15.

10. Procédé suivant l'une quelconque des revendications 1 à 9, dans lequel la température qui règne est comprise dans l'intervalle d'environ 100° à environ 300°C.

11. Procédé suivant l'une quelconque des revendications 1 à 10, dans lequel la pression est comprise dans l'intervalle d'environ 10 à 100 bars.

12. Procédé suivant l'une quelconque des revendications 1 à 11, dans lequel la réaction est conduite en l'absence de solvant inerte.

13. Procédé suivant l'une quelconque des revendications 1 à 11, dans lequel la réaction est conduite en présence d'un solvant inerte.

14. Procédé suivant la revendication 13, dans lequel ledit solvant est choisi entre des hydrocarbures aromatiques, nitriles, éthers, cétones, amides et esters.

15. Procédé suivant l'une quelconque des revendications 1 à 14, dans lequel de l'oxygène est présent dans le réacteur et le rapport CO:O₂ est compris dans l'intervalle d'environ 5:1 à 20:1.
